# EUROPEAN PATENT SPECIFICATION

(11) **EP 2 406 204 B1**
(45) Date of publication and mention of the grant of the patent: **06.09.2017**
(21) Application number: 10710475.4
(22) Date of filing: 11.03.2010
(51) Int. Cl.: C07C 7/10, C07C 7/12, C07C 11/02

(54) **OLEFIN FEED PURIFICATION PROCESS**
VERFAHREN ZUR AUFREINIGUNG EINES OLEFINSTROMS
PROCÉDÉ DE PURIFICATION D'UNE CHARGE OLÉFINIQUE

(30) Priority: 13.03.2009 US 210005 P; 23.03.2009 US 210843 P
(43) Date of publication of application: 18.01.2012
(73) Proprietor: ExxonMobil Research and Engineering Company, Annandale, NJ 08801-0900 (US)
(72) Inventor: KALYANARAMAN, Mohan, Media, PA 19063 (US); HOSKIN, Dennis, H., Westampton, NJ 08060 (US); CLARK, Michael, C., Chantilly, VA 20152 (US)
(74) Representative: ExxonMobil Chemical Europe Inc.
(86) International application number: PCT/US2010/000729
(87) International publication number: WO 2010/104579

(56) References cited:
- WO-A1-2007/104385
- US-A- 6 072 095
- US-A1- 2006 014 990

## Description

### Cross Reference to Related Applications

This application relates and claims priority to US Provisional Patent Application No. 61/210,005, filed on March 13, 2009 and US Provisional Patent Application No. 61/210,843, filed on March 23, 2009.

This application is related to applications Serial Nos. 11/362,257 and 11/362,128 claiming priority, respectively, from U.S. Applications Serial Nos. 60/656,954 and 60/656,947, now published as US 2006/0194999 and US 2006/0194995.

### Field of the Invention

This invention relates to a method of purifying the olefin feeds used in an olefin polymerization process and to the polymerization process with the feed purification.

### Background of the Invention

US 2006/0194999 describes a process for the production of high quality hydrocarbon fuels in the gasoline boiling range by the polymerization (actually, oligomerization although the term "polymerization" is often used in reference to the process) of low molecular weight olefins, principally olefins from FCC fuel gas, mainly ethylene and propylene and possibly butene. A development of the basis process is described in US 2006/0194995 where the oligomerization is combined in unit with a reformate alkylation step which reduces the benzene content of reformats and other light aromatic streams.

US6072095 discloses that after the coalescence purification a liquid organic fraction is obtained, as well as pressures from 100 to 2000 kPa during purification, purification of a feed comprising isobutene. The temperature used in the examples is 30 °C together with Lb1 (fraction obtained after the coalescence apparatus) being liquid.

In conventional olefin oligomerization processes the catalyst is a solid phosphoric acid catalyst made by sorbing phosphoric acid on kieselguhr but a significant improvement in the process is achieved as described in US 2006/0194999 by using a zeolite catalyst preferably of the MWW family. Both the conventional SPA catalyst and the zeolite catalysts are prone to poisoning by trace quantities (ppm levels) of contaminants such as acetonitrile (ACN), amines, sodium which may be present as carryover from the FCC or which are introduced during caustic and water wash steps used to purify the feed. The zeolite catalysts are more robust than the SPA catalyst but they nevertheless are sensitive to organic compounds with basic nitrogen as well as sulfur-containing organics. It is therefore preferred to remove these materials prior to entering the oligomerization unit if extended catalyst life is to be expected. Scrubbing with contaminant removal washes such as caustic, MEA (monoethanolamine) or other amines or aqueous wash liquids will normally reduce the sulfur level to an acceptable level of about 10-20 ppmw and the nitrogen to trace levels at which it can be readily tolerated. Although activity benefits are achieved by the use of low or very low water levels in the feed, the zeolite catalysts are not otherwise unduly sensitive to water, making it less necessary to control water entering the reactor than it is with SPA units. Unlike SPA, the zeolite catalyst does not require the presence of water in order to maintain activity and therefore the feed may be dried before entering the unit, for example, to below 200 ppmw water or lower, e.g. below 50 or even 20 ppmw. In conventional SPA units, the water content typically needs to be held between 300 to 500 ppmw at conventional operating temperatures for adequate activity while, at the same time, retaining catalyst integrity. The zeolite catalysts, however, may readily tolerate higher levels of water up to about 1,000 ppmw water although levels above about 800 ppmw may reduce activity, depending on temperature. Thus, with converted units, the olefin feed may contain from 300 or 500 to 1,000 ppmw water, although 300-800 ppmw should be regarded as a workable range for activity with existing feed treatment equipment.

The use of a guard bed prior to the oligomerization reactor may be desirable since the refinery feeds customarily routed to polymerization units (as distinct from petrochemical unit feeds which are invariably high purity feeds for which no guard bed is required) may have a contaminant content, especially of polar catalyst poisons, such as the polar organic nitrogen and organic sulfur compounds, which is too high for extended catalyst life. The use of a cheaper catalyst in the guard bed reactors which can be readily regenerated in swing cycle operation or, alternatively disposed of on a once-through basis, is normally viewed as desirable in ensuring extended cycle duration for the active polymerization catalyst.

While a water wash is effective to remove basic nitrogenous contaminants such as amines, large quantities may be necessary to achieve adequate feed purity presenting not only a problem in the wash units themselves but also in the disposal of the contaminated water after the wash. Another problem is that excess levels of water in the feed may depress the activity of zeolite catalysts or, in the case of SPA catalysts, result in disintegration and complete inactivation of the catalyst. For these reasons, feed purification and control of water content are required regardless of the catalyst actually used.

### Summary of the Invention

According to the present invention, the light olefin feed for an olefin utilization process is subjected to a water wash to remove water-soluble contaminants after which the water is separated from the olefin by coalescence separation at a low temperature, typically below 40°C. The coalescence separation technique has shown itself to be sufficiently effective and efficient that it may even be possible to carry out a supplemental washing by adding additional water to the feed/water mix after the initial wash step but before the coalescence step in order to remove more of the water-soluble contaminants. As an alternative to the coalescence technique, the water may be removed from the feed/wash mixture by contact it with a Super Absorbent Polymer (SAP) such as the sodium salt of a polyacrylic acid.

### Detailed Description

### Olefin Utilization Processes

The present olefin purification process may be applied to purifying the light olefin feeds to any process in which the feeds are to be utilized, typically in catalytic processes using a solid catalyst, especially a solid zeolite catalyst, The present purification process is particularly applicable to the purification of light olefin feeds which are to be used in oligomerization to higher hydrocarbons, in the alkylation of reformates and other aromatic feeds for example, in the production of cumene by the alkylation of benzene, the production of ethylbenzene by the ethylation of benzene with ethylene, the production of sec-butyl benzene by the alkylation of benzene with n-butene. Processes of this kind are described, for example in the following: U.S. Patent Publication No. 2006/0194999 (Olefin Oligomerization); U.S. Patent Publication No. 2006/0194998 (Reformate Alkylation); U.S. Patent Publication No. 2006/0194997 (Vapor Phase Reformate Alkylation); U.S. Patent Publication No. 2006/0194996 (Liquid Phase Reformate Alkylation); U.S. Patent Publication No. 2006/0194995 (Olefin Polymerization with Aromatics Alkylation); U,S. Patent 6888037 (Cumene Production) United States Patent No. 5493065 (Ethylbenzene Production) and U.S. Patent Publication No. 2008/0154082, (Production of sec-butyl benzene). These patents and publications cited here are purely exemplary and other processes for these and other reactions using light olefin feeds with these and other co-reactants and other catalysts are well-known; the application of the present olefin purification technique is generally applicable to all such processes.

### Olefin Feed

The light olefins used as the feed in the present olefin oligomerization process are normally obtained by the catalytic cracking of petroleum feedstocks to produce gasoline as the major product. The catalytic cracking process, usually in the form of fluid catalytic cracking (FCC) produces large quantities of light olefins as well as olefinic gasolines and by-products such as cycle oil which are themselves subject to further refining operations. The olefins which are primarily useful in the present process are the lighter olefins from ethylene up to butene (C₂-C₄); although the heavier C₅+ olefins may also be included in the processing, they can generally be incorporated directly into the gasoline product where they provide a valuable contribution to octane. The oligomerization process will operate readily not only with butene and propylene but also with ethylene and thus provide a valuable route for the conversion of this cracking byproduct to desired gasoline products. For this reason as well as their ready availability in large quantities in a refinery, mixed olefin streams such a FCC Off-Gas streams (typically containing ethylene, propylene and butenes) are used. Conversion of the C₃ and C₄ olefin fractions from the cracking process provides a direct route to the branch chain C₆, C₇ and C₈ products which are so highly desirable in gasoline from the view point of boiling point and octane. Besides the FCC unit, the mixed olefin streams may be obtained from other process units including cokers, visbreakers and thermal crackers. The presence of diolefins which may be found in certain refinery streams such as those from thermal cracking is not desirable in view of their tendency to form high molecular weight polymerization products which indicates that their removal in a diolefin saturation unit is preferred.

The compositions of two typical FCC gas streams is given below in Tables 1 and 2, Table 1 showing a light FCC gas stream and Table 2 a stream from which the ethylene has been removed in the gas plant for use in the refinery fuel system.

**Table 1**

| **FCC Light Gas Stream** | | |
|---|---|---|
| **Component** | **Wt. Pct.** | **Mol. Pct.** |
| Ethane | 3.3 | 5.1 |
| Ethylene | 0.7 | 1.2 |
| Propane | 14.5 | 15.3 |
| Propylene | 42.5 | 46.8 |
| Iso-butane | 12.9 | 10.3 |
| n-Butane | 3.3 | 2.6 |
| Butenes | 22.1 | 18.32 |
| Pentanes | 0.7 | 0.4 |

**Table 2**

| **C₃-C₄ FCC Gas Stream** | |
|---|---|
| Component | Wt. Pct. |
| 1- Propene | 18.7 |
| Propane | 18.1 |
| Isobutane | 19.7 |
| 2-Me-1-propene | 2.1 |
| 1-Butene | 8.1 |
| n-Butane | 15.1 |
| Trans-2-Butene | 8.7 |
| Cis-2-butene | 6.5 |
| Isopentane | 1.5 |
| C3 Olefins | 18.7 |
| C4 Olefins | 25.6 |
| Total Olefins | 44.3 |

### Oligomerization Catalyst

The present feed purification technique is generally applicable with olefin utilization processes using a solid, acidic catalyst which is preferably a molecular sieve catalyst, although an acidic amorphous catalyst such as SPA (solid phosphoric acid) will also function although less effectively than the preferred zeolite catalysts. Zeolites with 10-membered ring systems are preferred in these reactions, especially zeolites of the MEL, MFI, MFS, MTT, MTW, NU-87, MWW and TON structural types having the requisite degree of acidic functionality.

Zeolites of the MWW family are preferred for many of these reactions including olefin oligomerization, reformate alkylation, and for cumene and ethylbenzene production This family is currently known to include a number of zeolitic materials such as PSH-3 (described in U.S. Patent No. 4,439,405), MCM-22 (described in U.S. Patent No. 4,954,325), MCM-36 (described in U.S. Patent No. 5,250,277), MCM 49 (described in U.S. Patent No. 5,236,575), MCM 56 (described in U.S. Patent No. 5,362,697), SSZ 25 (described in U.S. Patent No. 4,826,667), ERB-1 (described in U.S. Patent No. EP 029302), ITQ-1 (described in U.S. Patent No. 6,077,498), ITQ-2 (described in WO 97/17290), UZM-8 (described in U.S. Patent No. 6,756,030). Of these, the four significant members of for use as olefin oligomerization catalysts are MCM-22, MCM-36, MCM-49, and MCM-56 with preference given to MCM-22 and MCM-49. It has been found that the MCM-22 or MCM-49 catalysts may be either used fresh, that is, not having been previously used as a catalyst or alternatively, regenerated catalyst may be used. Regenerated catalyst may be used after it has been used in any of the catalytic processes for which it is suitable, including the present process in which the catalyst has shown itself to remain active even after multiple regenerations. It may also be possible to use MWW catalysts which have previously been used in other commercial processes and for which they are no longer acceptable, for example, catalyst which has previously been used for the production of aromatics such as ethylbenzene or cumene, normally using reactions such as alkylation and transalkylation, as described in U.S. 2006/0194998. A full description of the MWW zeolites which are the optimal zeolite catalysts is given in US 2006/0194999, to which reference is made for a full description of these catalysts and the manner in which they may be used in the oligomerization of the light olefin feeds.

### Olefin Utilization Conditions

The olefin utilization reaction is carried out at the appropriate conditions for the reaction and its desired products. The olefin oligomerization reaction, for example, is carried out under conditions appropriate to the equipment and catalyst in use. For oligomerization using a MWW zeolite catalyst, suitable reaction conditions will be as described in as US 2006/0194999, to which reference is made for a full description of appropriate reactions conditions. Briefly, the process may be operated at low to moderate temperatures and pressures. In general, the temperature will be from about 120° to 250°C (about 250° to 480°F) and in most cases between 150° and 250°C (about 300°-480°C). Temperatures of 170° to 205°C (about 340° to 400°F) will normally be found optimum for feeds comprising butene while higher temperatures will normally be appropriate for feeds with significant amounts of propene. Pressures may be those appropriate to the type of unit so that pressures up to about 7500 kPag (about 1100 psig) will be typical but normally lower pressures will be sufficient, for example, below about 7,000 Kpag (about 1,000 psig) and lower pressure operation may be readily utilized, e.g. up to 3500 kPag (about 500 psig). Pressures of this order are consistent with those preferred for the water wash as noted below, therefore enabling the entire unit to be operated without de- or re-pressurization at any point. Ethylene, again, will require higher temperature operation to ensure that the products remain in the gasoline boiling range. Space velocity may be quite high, for example, up to 50 WHSV (hr⁻¹) but more usually in the range of 5 to 30 WHSV. Appropriate adjustment of the process conditions will enable co-condensation products to be produced when ethylene, normally less reactive than its immediate homologs, is included in the feed. Other olefin utilization reactions will be carried out at tier own respective appropriate conditions as described, for example, in the publications referred to above.

### Water Wash

The mixed light olefin feed is first subjected to a wash with an aqueous wash liquid which is typically caustic or plain water or both, depending on the contaminants known or suspected to be present. Caustic wash is especially effective to remove mercaptans and other sulfur impurities while water is effective for nitrogenous and other water-soluble bases. The ratio of water to olefin is typically in the range of 1:1 to 10 :1 by weight. Wash unit design will be conventional with the objective of ensuring good contact between the olefin feed and the water with various types of contactor applicable, for example, scrubbers, countercurrent towers, During this step and the following coalescence step, the conditions should be chosen so as to maintain the olefin stream in the liquid phase since this will favor removal of the contaminant species. The preferred temperatures for the feed and the wash water below about 40°C and preferably below 35°C, since water tends to be more soluble in the feed at higher temperatures and so tends to dissolve in the feed and be less amenable to separation by coalescence and, remaining in the feed will take with it the water soluble contaminants such as acetonitrile and nitrogenous bases. Thus, operation of the water wash and the subsequent coalescence not only reduces the water content of the feed, in itself desirable, but also reduces the level of impurities in the feed. Operation at temperatures below about 25 or 20°C can be desirable from this point of view. At the preferred temperatures, the olefins will be in the liquid phase at pressures of about 2100-4200 kPa (approx. 300-600 psia.).

A supplemental wash step may follow the initial wash to help dissolve soluble poisons prior to separation of the water. The amount of water used in this step relative to the feed is typically from about 0.5:1 to 2:1 by weight.

### Coalescence Separation

Following the water wash the hydrocarbon feed is separated from the water by means of a coalescer separator. The method of coalescing a liquid suspended in another immiscible phase using a coalescer, has been found useful for removing liquids both from the gaseous phase as in aerosols and from suspensions of one liquid in another liquid with which it is immiscible but may be soluble to a limited degree. The coalescence separation technique has become commercially attractive in recent years for separating immiscible liquids especially hydrocarbons and water. See, for example, *Refining Details: Advances in Liquid*/*Liquid Coalescing Technology,* Gardner, Today's Refinery, March 1997. Coalescing devices are particularly effective where the volume of liquid to be removed is small in comparison to the volume of the phase from which it is removed. The Gardener article discusses the factors that are relevant to the coalescence of droplets of the discontinuous phase from the continuous phase and the ease or difficulty of separation of the immiscible phases. These factors include the physical properties of the phases such as density, viscosity, surface tension and interfacial tension. In addition, the properties of the system such as drop size, curvature of the liquid/liquid interface, temperature, concentration gradients and vibrations may also affect the effectiveness of the coalescence. As noted in U.S. Patent No. 5, 443,724 (Williamson), any or all of these factors may be significant in a particular situation but the density, drop size and interfacial tension of the two liquids appear to be the most significant factors as well as those over the least amount of control can be exercised in affecting the separations.

The liquid-liquid coalescence typically operates in three stages, as noted by Hampton in Liquid-Liquid Separation Technology, Oil & Gas Journal, 16 April 2001, 54-57, namely separation of solids, coalesence and separation of coalesced droplets..

Prefilters which are recommended for use ahead of the coalescer may be any suitable type of conventional filter, including sand filters, metal or polymer meshes, or other porous material capable of removing small solid particles which would tend to stabilize hydrocarbon/water emulsions and which might result in damage to the more delicate coalescer membranes. Polyester and nylon mesh filters are suitable, typically with crush strengths in the range of 70-145 kg.cm⁻² (75 - 150psi) and other non-woven filter materials may be used as convenient alternatives. The filter material may be contained in a conventional filter housing and the filter material in any convenient configuration which provides the desired filter life, filtration capacity and flow rate, for example, pleated mats, cylindrical sheets or mats, helical or spirally wound mats.

In a similar manner, the material of the coalescer and separation elements in the coalescing unit and the separation unit may be provided in a form which provides the necessary mechanical strength, liquid flow rate and unit life. In the simplest form, the media serving as the coalescer and separator materials may be provided in sheet form which may be formed either as flat sheets, pleated or corrugated sheets or in other suitable arrangements e.g. cylindrically, helically or spirally wound sheets, as disclosed in U.S. 5,443,724 to which reference is made for a disclosure of suitable coalescer and separator materials and configurations for them. Coalescers should be selected for capability of operation at the pressures preferred for the water wash (2100-4200 kPa).

The coalescer promotes the coalescence of the discontinuous or highly divided phase of the water/olefin mixture in the form of finely divided water droplets into larger and coarser droplets. The pore structure of the coalescer begins with a very fine structure and then opens up to provide void space for the coalescing droplets. The coalescence mechanism has been stated (Hampton, *op cit.*) to involve the adsorption of droplets by the fibers of the coalescence material followed by translation along the fibers and collisions at the junctures of fibers. The collisions result in droplets merging or coalescing and the viscous drag of the bulk fluid stream then causes the enlarged drops to disengage from the fibers. This process is repeated several times through the coalescer medium until the large, coalesced drops leave the coalescer medium. Once coalesced, the droplets are assumed to be as large as possible for the given flow conditions. Separation them removes the drops from the main body of liquid and the coalesced droplets collected and removed from the olefins in a gravity separator, e.g. a knock out drum.

The coalescing material is used in the form of a packing formed of a material which has a critical wetting surface energy intermediate the surface tensions of the liquids forming the continuous and discontinuous phases, that is, of the hydrocarbon component and the water. Similarly, the material of the separating element is selected so as to have a surface energy which permits passage of the coalesced droplets made up of extractant plus removed hydrocarbon through the small pores of the separator material but to preclude transfer of the water across the wall. As disclosed in U.S. 5,443,724, materials preferred for use as the phase barrier material for the separator include silicones, such as silicone treated paper and more preferably fluoropolymeric materials of which fluorocarbons or perfluorocarbons (perfluoro resins) are particularly preferred. Examples of preferred materials for use as the packing or coating in the separator include polytetrafluoroethylene (PTFE) or other polyfluorinated polymers such as fluorinated ethylenepropylene (FEP) resins. As noted, a preferred separator material includes a coating of one of these materials on a stainless steel screen or a pleated paper pack. Other suitable materials include those disclosed in U.S. 4,759,782 to which reference is made for a disclosure of such materials. Generally, the phase barrier material which acts to prevent the discontinuous phase passing through it (and is therefore appropriately referred to as the discontinuous phase barrier material) is selected to have pores smaller than a substantial amount of the droplets of the liquid which forms the discontinuous phase. Typically, the pore size of the functional part of the separator material is selected to be from 5 to 140 microns, preferably 40 to 100 microns.

The coalescing unit and the separation unit may suitably be contained in a housing which provides and adequate number of coalescing/separating elements with these elements being suitably arranged inside the housing for reasons of functionality and operating convenience. A suitable arrangement is shown in U.S. 5,443,724, using coalescer and separator cartridge elements arranged in super posed relationship with one another in a cylindrical type housing which permits ready access to the cartridges when they require replacement. However, other configurations may be used and reference is made to commercial suppliers of this equipment including Pall Corporation of East Hills, NY 11548.

As noted above, the temperature should be held should be held below about 40°C and preferably below 35°C in order to reduce the solubility of the water in the feed, with operation at temperatures below about 25 or 20°C can be desirable from this point of view. After the wash step, a lot of free water remains in the feed and when the feed is subsequently heated up to reaction temperature, much of the free water becomes dissolved in the feed and carries the water soluble impurities with it into contact with the catalyst. Thus, operation of the separation at low temperature favors elimination of the water from the mixture ultimately fed to the oligomerization reactor. Options for low temperature operation include cooling of the feed/water mixture by passage through a heat exchanger prior to entry into the coalescer or, alternatively, the coalescer unit or cartridge can be housed in a cool chamber at the desired temperature. However achieved, the reduction in temperature will result in a reduction of the contaminant level in the olefin feed as a result of the lower solubility of the water with its dissolved impurities in the hydrocarbon feed.

As noted above, the water may alternatively be removed from the feed/wash mixture by contact it with a Superabsorbent Polymer (SAP). Superabsorbent polymers (also called slush powder) are polymers that can absorb and retain extremely large amounts of a liquid relative to the mass of the polymer used. Superabsorbing polymers which take up water, classified as hydrogels, absorb aqueous solutions through hydrogen bonding with the water molecule. So an SAP's ability to absorb water is a factor of the ionic concentration of the aqueous solution with which it is in contact. In deionized and distilled water, SAP may absorb 500 times its weight (from 30-60 times its own volume), but when put into a 0.9% saline solution, the absorbency drops to maybe 50 times its weight.

Superabsorbent polymers are commonly made from the polymerization of acrylic acid blended with sodium hydroxide in the presence of an initiator to form a polyacrylic acid, sodium salt, sometimes referred to as cross-linked sodium polyacrylate. Other materials are also used to make a superabsorbent polymer, such as polyacrylamide copolymer, ethylene maleic anhydride copolymer, cross-linked carboxy-methyl-cellulose, polyvinyl alcohol copolymers, cross-linked polyethylene oxide, and starch grafted copolymer of polyacrylonitrile. Superabsorbent polymers may be made by either the suspension polymerization or solution polymerization method. The total absorbency and swelling capacity of the polymer are controlled by the type and degree of cross-linking to the polymer. Low density cross-linked SAP generally has a higher absorbent capacity and swell to a larger degree. These types of SAPs also have a softer and more cohesive gel formation. High cross-link density polymers exhibit lower absorbent capacity and swell. The gel strength is firmer and can maintain particle shape even under modest pressure.

The feed/wash water mixture is brought into contact with the SAP after completion of the washing step or steps and the aqueous phase removed from the hydrocarbon phase by absorption. Given the hydrophilic nature of the SAP, the hydrocarbon olefin phase is not taken up by the polymer and therefore is relatively free of the contaminants dissolved in the water. The polymer can be contained in any suitable dryer vessel for this step.

It is contemplated that the present invention may be used in connection with sPA catalysts by first removing contaminants through a water wash and removal of the contaminated water by coalescence followed by injection of clean water at desired levels in order to maintain activity of sPA catalyst. Thus, it is intended that the present invention covers the modifications and variations of the methods herein, provided they come within the scope of the appended claims.

## Claims

1. A process for purifying a mixed light olefin feed for an olefin utilization process which comprises subjecting the feed to a water wash to remove water-soluble contaminants and separating the washed feed from the water by coalescence separation at a temperature below 40°C, wherein the water wash is carried out at a temperature below 40°C and at a pressure of 2100-4200 kPa with the olefin feed in the liquid phase, and wherein the mixed light olefin feed comprises olefins from ethylene up to butene.

2. A process according to claim 1 in which the water wash is carried out at a temperature below 35°C

3. A process according to claim 1 in which wash water is separated from the olefin feed subsequent to the coalescence step by gravity separation.

4. A process according to claim 1 in which wash water is separated from the olefin feed prior to the coalescence step by gravity separation.

5. A process according to claim 1 in which the feed is washed with additional wash water after the initial wash and prior to the coalescence step.

6. A process of any of the preceding claims in which the olefin utilization process is a reaction over a catalyst.

7. A process according to claim 6 in which the catalyst is a solid zeolite catalyst.

8. A process according to claim 6 in which the catalyst is an sPA catalyst.

9. A process according to claim 6 in which the olefin utilization process is olefin oligomerization.

10. A process according to claim 9 in which the light olefin feed is a C2-C4 olefin feed, the oligomerization is carried out at a temperature of 120 to 250°C and a pressure of up to 7,000 kPa.

11. A process according to claim 6 in which the olefin utilization process is reformate alkylation.

12. A process according to claim 6 in which the olefin utilization process is alkylation of a benzene feed to make cumene or ethylbenzene.

13. A process for purifying a mixed light olefin feed for an olefin oligomerization process which comprises subjecting the feed to a water wash to remove water-soluble contaminants and separating the washed feed from the water by contact with a superabsorbent polymer a temperature below 40°C, wherein the water wash is carried out at a temperature below 40°C and at a pressure of 2100-4200 kPa with the olefin feed in the liquid phase, and wherein the mixed light olefin feed comprises olefins from ethylene up to butene.

14. A process according to claim 13 in which the superabsorbent polymer comprises a cross-linked sodium polyacrylate, an polyacrylamide copolymer, an ethylene maleic anhydride copolymer, a cross-linked carboxy-methyl-cellulose, a polyvinyl alcohol copolymer, a cross-linked polyethylene oxide, or a starch grafted copolymer of polyacrylonitrile.

15. An olefin oligomerization process comprising a process of purifying a mixed light olefin feed according to any one of claims 1 to 14 and oligomerizing the purified mixed light olefin feed over a catalyst.

## Patentansprüche

1. Verfahren zum Reinigen eines gemischten leichten Olefineinsatzmaterials für ein Olefin-Verwertungsverfahren, bei dem das Einsatzmaterial mit Wasser gewaschen wird, sodass wasserlösliche Verunreinigungen entfernt werden, und das gewaschene Einsatzmaterial von dem Wasser mit Hilfe einer Koaleszenztrennung bei einer Temperatur unterhalb von 40°C abgetrennt wird, wobei das Waschen mit Wasser bei einer Temperatur unterhalb von 40°C und einem Druck von 2100 bis 4200 kPa durchgeführt wird, wobei das Olefineinsatzmaterial in der flüssigen Phase ist, und bei dem das gemischte leichte Olefineinsatzmaterial Olefine von Ethylen bis hoch zu Buten umfasst.

2. Verfahren nach Anspruch 1, bei dem das Waschen mit Wasser bei einer Temperatur unterhalb von 35°C durchgeführt wird.

3. Verfahren nach Anspruch 1, bei dem Waschwasser nach der Koalezenzstufe durch Schwerkrafttrennung von dem Olefineinsatzmaterial abgetrennt wird.

4. Verfahren nach Anspruch 1, bei dem Waschwasser vor der Koalezenzstufe durch Schwerkrafttrennung von dem Olefineinsatzmaterial abgetrennt wird.

5. Verfahren nach Anspruch 1, bei dem das Einsatzmaterial mit zusätzlichem Waschwasser nach dem anfänglichen Waschen und vor der Koalezenzstufe gewaschen wird.

6. Verfahren nach einem der vorhergehenden Ansprüche, bei dem das Olefin-Verwertungsverfahren eine Reaktion über einem Katalysator ist.

7. Verfahren nach Anspruch 6, bei dem der Katalysator ein Festkörper-Zeolithkatalysator ist.

8. Verfahren nach Anspruch 6, bei dem der Katalysator ein sPA-Katalysator ist

9. Verfahren nach Anspruch 6, bei dem das Olefin-Verwertungsverfahren eine Olefin-Oligomerisierung ist.

10. Verfahren nach Anspruch 9, bei dem das leichten Olefinein satz material ein C₂- bis C₄-Olefineinsatzmaterial ist, die Oligomerisierung bei einer Temperatur von 120 bis 250°C und einem Druck von bis zu 7000 kPa durchgeführt wird.

11. Verfahren nach Anspruch 6, bei dem das Olefin-Verwertungsverfahren Reformatalkylierung ist.

12. Verfahren nach Anspruch 6, bei dem das Olefin-Verwertungsverfahren die Alkylierung von Benzoleinsatzmaterial zur Herstellung von Cumol oder Ethylbenzol ist.

13. Verfahren zum Reinigen eines gemischten leichten Olefineinsatzmaterials für e i n Olefin-Oligomerisierungsverfahren, bei dem das Einsatzmaterial mit Wasser gewaschen wird, sodass wasserlösliche Verunreinigungen entfernt werden und das gewaschene Einsatzmaterial von dem Wasser durch Kontakt mit einem Superabsorberpolymer bei einer Temperatur unterhalb von 40°C abgetrennt wird, wobei das Waschen mit Wasser bei einer Temperatur unterhalb von 40°C und einem Druck von 2100 bis 4200 kPa durchgeführt wird, wobei das Olefineinsatzmaterial in der flüssigen Phase ist, und bei dem das gemischte leichte Olefineinsatzmaterial Olefine von Ethylen bis hoch zu Buten umfasst.

14. Verfahren nach Anspruch 13, bei dem das Superabsorberpolymer vernetztes Natriumpolyacrylat, Polyacrylamid-Copolymer, Ethylenmaleinsäureanhydrid-Copolymer, vernetzte Carboxyl-Methyl-Cellulose, Polyvinylalkohol-Copolymer, vernetztes Polyethylenoxid oder mit Stärke gepfropftes Copolymer aus Polyacrylnitril umfasst.

15. Olefin-Oligomerisierungsverfahren, das ein Verfahren umfasst, bei dem ein gemischtes leichtes Olefineinsatzmaterial gemäß einem der Ansprüche 1 bis 14 gereinigt wird und das gereinigte gemischte leichte Olefineinsatzmaterial über einem Katalysator oligomerisiert wird.

## Revendications

1. Procédé de purification d'une charge d'oléfines légères mélangées pour un procédé d'utilisation d'oléfines qui comprend la soumission de la charge à un lavage à l'eau pour retirer les contaminants solubles dans l'eau et la séparation de la charge lavée de l'eau par séparation par coalescence à une température inférieure à 40 °C, le lavage à l'eau étant réalisé à une température inférieure à 40 °C et à une pression de 2100-4200 kPa avec la charge d'oléfines dans la phase liquide, et la charge d'oléfines légères mélangées comprenant des oléfines allant de l'éthylène au butène.

2. Procédé selon la revendication 1 dans lequel le lavage à l'eau est réalisé à une température inférieure à 35 °C.

3. Procédé selon la revendication 1 dans lequel l'eau de lavage est séparée de la charge d'oléfines après l'étape de coalescence par séparation par gravité.

4. Procédé selon la revendication 1 dans lequel l'eau de lavage est séparée de la charge d'oléfines avant l'étape de coalescence par séparation par gravité.

5. Procédé selon la revendication 1 dans lequel la charge est lavée avec de l'eau de lavage supplémentaire après le lavage initial et avant l'étape de coalescence.

6. Procédé de l'une quelconque des revendications précédentes dans lequel le procédé d'utilisation d'oléfines est une réaction sur un catalyseur.

7. Procédé selon la revendication 6 dans lequel le catalyseur est un catalyseur zéolithique solide.

8. Procédé selon la revendication 6 dans lequel le catalyseur est un catalyseur sPA.

9. Procédé selon la revendication 6 dans lequel le procédé d'utilisation d'oléfines est une oligomérisation d'oléfines.

10. Procédé selon la revendication 9 dans lequel la charge d'oléfines légères est une charge d'oléfines en C2-C4, l'oligomérisation est réalisée à une température de 120 à 250 °C et une pression allant jusqu' à 7000 kPa.

11. Procédé selon la revendication 6 dans lequel le procédé d'utilisation d'oléfines est une alkylation de reformat.

12. Procédé selon la revendication 6 dans lequel le procédé d'utilisation d'oléfines est l'alkylation d'une charge de benzène pour fabriquer du cumène ou de l'éthylbenzène.

13. Procédé de purification d'une charge d'oléfines légères mélangées pour un procédé d'oligomérisation d'oléfines qui comprend la soumission de la charge à un lavage à l'eau pour retirer les contaminants solubles dans l'eau et la séparation de la charge lavée de l'eau par contact avec un polymère superabsorbant à une température inférieure à 40 °C, le lavage à l'eau étant réalisé à une température inférieure à 40 °C et à une pression de 2100-4200 kPa avec la charge d'oléfines dans la phase liquide, et la charge d'oléfines légères mélangées comprenant des oléfines allant de l'éthylène au butène.

14. Procédé selon la revendication 13 dans lequel le polymère superabsorbant comprend un polyacrylate de sodium réticulé, un copolymère de polyacrylamide, un copolymère éthylène-anhydride maléique, une carboxy-méthylcellulose réticulée, un copolymère d'alcool polyvinylique, un oxyde de polyéthylène réticulé, ou un copolymère de polyacrylonitrile greffé à l'amidon.

15. Procédé d'oligomérisation d'oléfines comprenant un procédé de purification d'une charge d'oléfines légères mélangées selon l'une quelconque des revendications 1 à 14 et l'oligomérisation de la charge d'oléfines légères mélangées purifiée sur un catalyseur.
